Europäisches Patentamt

European Patent Office

Office européen des brevets

(19)

(11) EP 0 269 147 B1

(12) **EUROPEAN PATENT SPECIFICATION**

(45) Date of publication and mention
of the grant of the patent:
**21.02.1996 Bulletin 1996/08**

(51) Int Cl.6: **G01R 33/48**, G01R 33/56,
G06F 17/00, A61B 5/055

(21) Application number: 87202021.9

(22) Date of filing: **20.10.1987**

(54) **A time-clustered cardio-respiratory encoder and method for clustering cardio-respiratory signals**

Zeitgruppiertes Kodierungsgerät für Herz- und Atmungssignale und Methode zum Gruppieren derselben

Encodeur de signaux cardio-respiratoires groupés dans le temps et méthode pour grouper des signaux cardio-respiratoires

(84) Designated Contracting States:
**DE FR GB**

(30) Priority: **22.10.1986 US 921980**

(43) Date of publication of application:
**01.06.1988 Bulletin 1988/22**

(73) Proprietor:
**PHILIPS ELECTRONICS NORTH AMERICA
CORPORATION
New York, N.Y. 10017 (US)**

(72) Inventor: **Bohning, Daryl Eugene
NL-5656 AA Eindhoven (NL)**

(74) Representative: **Schouten, Marcus Maria et al
NL-5656 AA Eindhoven (NL)**

(56) References cited:
**US-A- 4 545 384**

• **IEEE TRANSACTIONS ON NUCLEAR SCIENCE,
vol. NS-31, no. 1, February 1984, pages
566-569, IEEE, New York, US; I.G. ZUBAL et al.:
"Dual gated nuclear cardiac images"**
• **PROCEEDINGS OF MELECON' 85
MEDITERRANEAN ELECTROTECHNICAL
CONFERENCE, Madrid, 8th-10th October 1985,
vol. II, Digital Signal Processing, Elsevier
(North Holland), pages 217-220, IEEE, New
York, US; Z. BJELOGRLIC et al.: "3-D analysis
of the left ventricular wall motion by clustering
technique"**

## Description

### BACKGROUND OF THE INVENTION.

#### Field of the Invention.

This invention pertains generally to the field of nuclear magnetic resonance (NMR) tomography and in particular to retrospective clustering and analysis of NMR k-space measurements in the cardiac phase - respiratory phase plane. The processing system and method effect the reduction of cardio-vascular and respiratory flow motion artifacts in images and the quantitative characterization of cardiac structure and function both with and without imaging.

#### Description of the Prior Art.

NMR spectroscopy, the process of analyzing a small sample in a uniform magnetic field and obtaining radio frequency data resulting from precisely pulsed radio frequency excitation, were invented by Bloch and Purcell. In the past 16 years, NMR analysis by spectroscopy has shifted from physical chemistry to biological chemistry and biological medical applications; i.e., biopsy samples of normal and diseased tissues. Lauterbur and Damadian and others separately invented the utilization of NMR principles to produce and image. (R. Damadian, Science 171,1151, 1971; P.C. Lauterbur, Nature 242, 190, 1973 and P.C. Lauterbur, Pure and Applied Chemistry, 40, 149, 1974). The resulting devices, NMR imaging systems, produced two deminsional and three dimensional data in which the gray scale represented is a function of a number of parameters, including for example the three parameters nuclide density, $T_1$ (longitudinal relaxation time) and $T_2$ (transverse relaxation time) especially in an anatomical image.

NMR imaging techniques are disclosed, for example, in "Proton NMR Tomography" P.R. Locher, Philips Technical Review, Vol. 41, 1983/84, No. 3, pages 73-88, the contents of which are referred to herein as a background of NMR imaging technology.

In vivo NMR imaging of biological tissue is rendered more difficult by movement of the biological tissue. This is especially true for example, in cardiac NMR imaging, as a result of the heartbeat. In order to prevent blurring and movement artifacts in cardiac NMR imaging, it is known to synchronize the measurements to the patients E.C.G., for example, as disclosed in U.S. Patent No. 4,409,550, Fossel et al and U.S. Patent No. 4,413,233, Fossel et al.

The noninvasive character of nuclear magnetic resonance (NMR) imaging and the absence of obscuration by bone structures makes it a desirable technique for heart imaging. The relatively long scan times needed, however, give rise to motion artifacts. Synchronization of the imaging sequences to the heart cycle can greatly reduce these artifacts.

The capability of imaging in any phase of the cardiac cycle makes it possible to use NMR for volumetric measurements from end-systole and end-diastole images. Also, the evaluation of motion is possible by displaying images from consecutive phases in a movie loop. The use and benefits of NMR imaging for displaying the anatomy of the heart and great vessels, both untriggered and electrocardiogram (ECG) triggered is well known. Apart from showing fine anatomical details in the heart, NMR heart imaging holds promise to tissue characterization as well, important for the detection and sizing of infarcts. Using velocity images, heart wall motion and blood flow speeds can also be quantitated with NMR imaging.

In the two-dimensional Fourier transform (2DFT) imaging method used, the NMR image is reconstructed from time signals by a complex 2DFT. For a 128 x 128 pixel image matrix, the signals are obtained in 128 consecutive imaging sequences. For heart imaging, each sequence is triggered by a pulse derived from the R-wave of the patient's ECG. The delay of this pulse determines the imaging phase in the cardiac cycle. Each sequence consists of a series of radiofrequency (RF) and magnetic field gradient pulses, respectively, for evoking a signal and providing spatial information in the signal. A gradient magnetic field applied after an RF excitation pulse (90° pulse) makes the spins at different locations in the excited slice precess at different frequencies. The spins start to dephase with respect to the resonant frequency phase. For a given location the associated phase shift is proportional to the gradient amplitude and the time it is active. There is, in addition, a difference in phase shift for stationary spins and moving spins. For spins moving uniformly in the direction of the gradient, an extra term is added that is directly proportional to the velocity in the gradient direction.

The present invention pertains to an improved method and system for correlating NMR data with data from cardiac/respiratory monitors commonly used for conventional cardiac triggering and respiratory gating. Many approaches to this problem have been tried in the prior art. NMR data collection has been started at fixed delays after the ECG R-wave, with possible rejection of abnormal R-R intervals. Typically, in the known arrangements, each data collection is started at some fixed time delay after the R-peak. In a multiple slice study, data collection continues close to the next R-peak. If the heart beats regularly, each measurement will take place at the same phase in the cardiac cycle.

In the event of arrhythmia, however, deviations of the heart's position will occur for some of the data collection, thereby giveing rise to blurring and movement artifacts. Such arrhythmia will further lead to a variation in the repetition time TR, resulting in an increased noise level and degradation of the precision of the $T_1$ measurements, as well as degrading its general image quality of the primary images, as noted by C. Galonad, D.J. Drost, S.S. Prato and G. Wisen berg, SMRM, Vol. 2, 1985. It is well known in nuclear medicine that rejection of data collection taken during and immediately after an arrhythmia improves image quality and consistency. In the past, however, it has been difficult to effect such data in a simple and efficacious manner.

Respiratory cycle timed NMR k-space sampling has also been utilized. In US-A-4,545,384 a method for sampling of k-space with respect to the respiratory cycle is disclosed. In this method NMR data are sampled synchronously with a selected position of the slice in the respiratory cycle, wherein the position of the slice in the respiratory cycle is derived from the amplitude of the NMR-signal. Subsequently an image of the slice in the selected position of the respiratory cycle can be reconstructed from the sampled data. However, discarding abnormal physiological cycles, or physiological cycle correlated sampling of k-space may actually cause abnormalities to be missed, which would defeat the diagnostic purpose of an NMR scan. Because of R-R variation, cardiac triggering is less effective at the end of a heart cycle when coronaries fill and the pre-R-wave shape of the heart may give clues to contraction abnormalities.

The prior art approaches tend to be complicated combinations of slices, phases, triggers, delays, windows and data rejection and reordering imposed on the NMR data collection, causing problems with uncertain heart phasing, limited time resolution, and nonuniform spin saturation. The present invention is designed to overcome these problems.

## SUMMARY OF THE INVENTION.

The time-clustered cardio-respiratory encoder of the present invention is a microcomputer based system which collects cardiac (C) R-wave time, respiratory (R) diaphragm position, and NMR data acquisition (A) timing data in parallel with free running image collection on a standard magnetic resonance imager. After the raw data equivalent of multiple images has been collected, the C-A-R phase timing data are uploaded from a microcomputer to the minicomputer of the scanner for use in clustering the raw data into a new data set, one image equivalent for each cardio-respiratory (C-R) phase combination desired. The position of an NMR profile in the normalized C-R phase plane determines the clustered image in which it will fall, i.e. which cardiac phase-respiratory phase combination. The clustered raw data are then filled and filtered to compensate for the nonuniform k-space sampling, and, finally, reconstructed with ordinary 2D or 3D Fourier Transform Techniques.

A method of processing image data with respect to a cardiac-respiratory cycle is known *per se* in a publication Dual Gated Nuclear Cardiac Images of I.G. Zubal et al, published in IEEE Transactions in Nuclear Science, Vol NS-31, No 1, February 1984. In this method, related to nuclear medicine, data at full expiration are selected and separated in 8 bins distributed of the heart cycle, each bin corresponding to a different heart phase. Data at corresponding phases in different cycles is then summed for better resolution. Accordingly, the combination of data is applied to improve resolution. The method according to the present invention relates to NMR, which is a completely different technique. In the method sufficient NMR-data is sampled, independent of the Cardiac-Respiratory (C-R) phase to fill the k-space several times. Simultaneously with each profile the C-R phase is recorded. Subsequently, MR data are combined belonging to the same C-R phase. The resulting k-space is filled in a non-uniform way. Filling and filtering of the raw data is then applied to compensate, whereafter an image can be reconstructed.

To obtain two multiple profile images, intended to show the heart at two different phases in its cycle, using the present invention one can obtain a time clustered image showing, for example, the first fifth of the cardiac cycle from all the profiles taken during the first fifth of their respective R-R interval and a time clustered image showing the fourth fifth of the cardiac cycle from the profiles taken during the fourth fifth of their respective R-R interval.

The system is easily implemented with any NMR system and can be used with any pulse sequence. It requires only readily available output signals from the standard cardiac/respiratory monitors commonly used for conventional cardiac triggering and respiratory gating, and software parameter modifications to accommodate different pulse sequences, along with a timing signal from the scanner.

The object of the invention is a simple, flexible system for retrospectively clustering and analyzing NMR k-space measurement profiles in the cardiac phase-respiratory phase plane to: 1) reduce cardio-vascular and respiratory flow/motion image artifacts, and 2) to quantitatively characterize cardiac structure/function both with and without imaging.

## BRIEF DESCRIPTION OF THE DRAWINGS.

Figure 1 is a functional block diagram showing the time clustered cardio-respiratory encoder of the present invention within a cardiac diagnostic system;

Figure 2 is a hardware block diagram of the time clustered cardio-respiratory encoder of the invention within an NMR cardiac diagnostic system;

Figure 3 is a functional block diagram of the method steps used in the time clustered cardio-respiratory encoder of the present invention;

Figure 4A is a plot showing the relationship of time clustered images to cardiac triggered images;

Figure 4B is a two dimensional clustering array in the normal cardiac and respiratory cycle plane.

## BRIEF DESCRIPTION OF THE PREFERRED EMBODIMENT.

The invention pertains to a method and its implementation in an encoder for retrospectively clustering NMR k-space measurement profiles in the cardiac phase respiratory phase plane, which method and encoder function to: 1) reduce cardio-vascular and respiratory flow motion, image artifacts, and 2) to characterize quantitatively cardiac structure/function both with and without imaging.

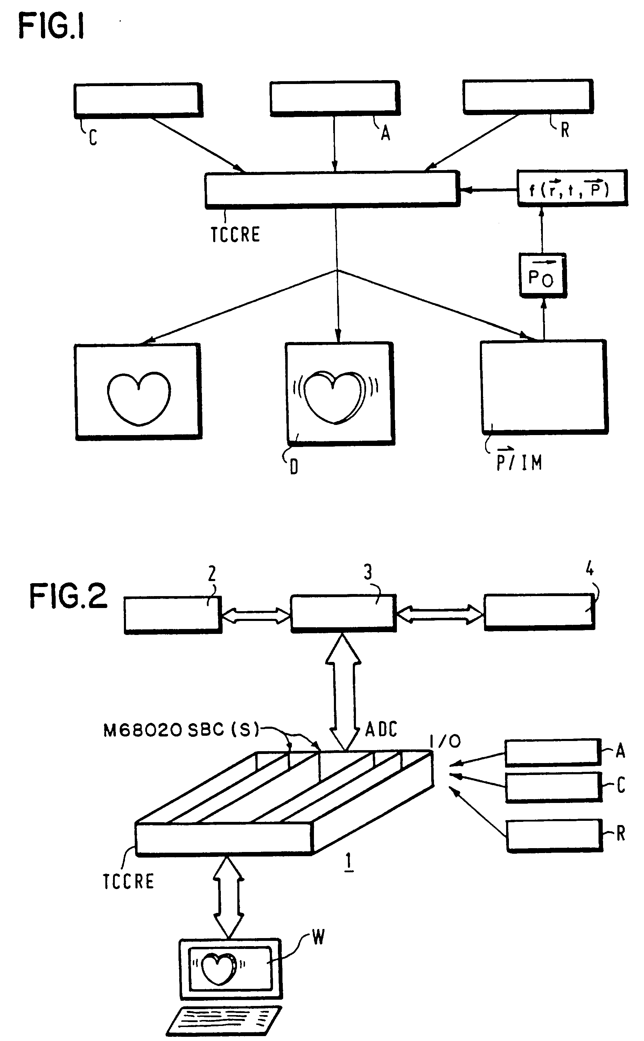

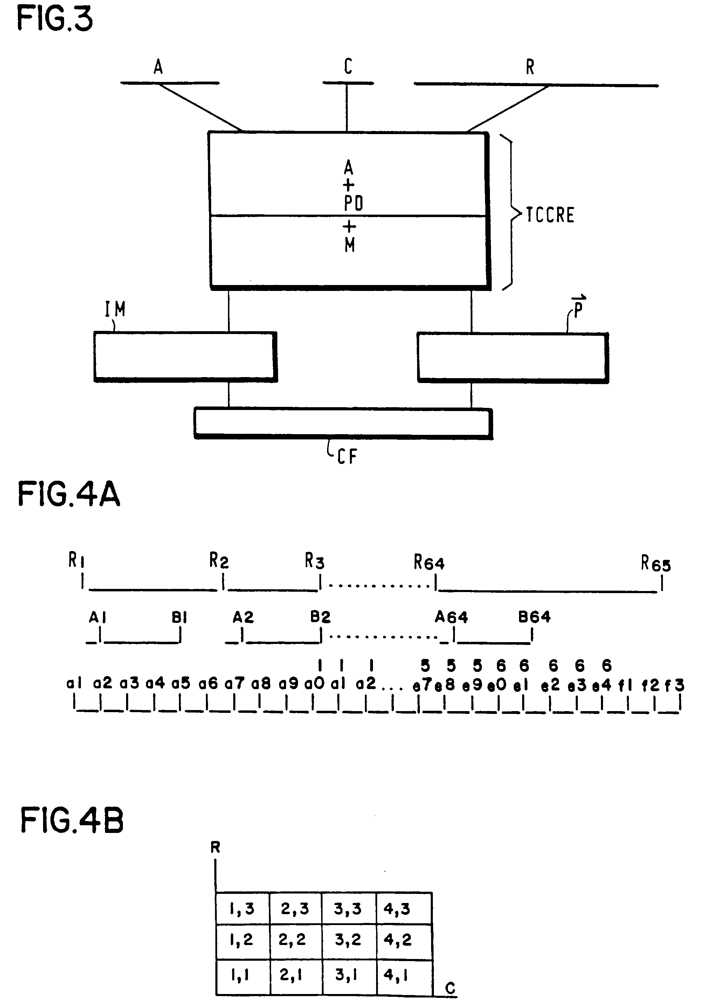

Figure 1 presents an overview of the time clustered cardio-respiratory encoder (TCCRE) of the present invention, while Figure 2 illustrates the hardware used and Figure 3 emphasizes the functional use of the encoder and the method. Referring to the figures, cardiac (C) time, respiratory (R) time of diaphragm position, and NMR data acquisition (A) cycle marker data are collected by a microcomputer (1) in parallel with free running, preferably steady state, image collection on a standard magnetic resonance imager (2). After collecting the raw data equivalent of multiple images, the C-A-R phase marker data are uploaded from the microcomputer to the minicomputer (3) of the NMR scanner for use in C-R plane clustering of the image raw data. The position of the NMR profile in the normalized C-R phase plane is determined and then clustered into a new set of data, one image equivalent for each desired C-R phase combination. These raw data are then filled and filtered to compensate for the nonuniform k-space sampling, and finally reconstructed.

The method of the present invention as illustrated in Figures 1 and 3 is as follows:

1. Collect NMR profiles from a free running, possibly velocity encoded pulse sequence, simultaneously collecting digitized ECG signal:
$$P (k\_x, k\_y, k\_z, t)$$
and
$$ECG ( t )\rightarrow ECG\_j (t); j\_th \text{ heart cycle.}$$
2. Use a technique, similar to that used for serial nuclear medicine data, to sort the signal profiles into normalized heart cycle time bins appropriate to the available signals to noise, i.e. using time of the profile minus the time of the previous R-wave, all divided by the time of the following R-wave minus the time of the previous R-wave to determine the time bin in which the profile should be put. The profile position in the respiratory cycle is determined the same way
$$P\_i ( k\_x, k\_y, k\_z, t ) ; i\_th \text{ phase of heart cycle } t\_i-1 < t < t\_i.$$
These data form a set of (velocity) images, one for each heart cycle phase time bin, selected for moving heart tissue, but each image of the set possibly covering k-space incompletely, and a little differently.
3. Fit the data to a parameterized dynamic heart model, while still in frequency space.
4. Fill, filter and transform this data using a discrete, nonuniform, Fourier Tranform to overcome the nonuniform k-space coverage.

5. Display if desired a dynamic simulation (D) of the heart, along with its parameters, on an integrated graphics workstation (W).
6. Update, if desired, the dynamic heart model using the newly reconstructed data.

TCCRE images show less flow/motion artifact and better heart visualization than comparably phased triggered images, especially towards the critical latter part of the cardiac cycle where coronary artery filling occurs.

As shown in Figures 1 and 3, the TCCRE receives digital cardiac R-wave signals, analog or digital respiratory input and NMR data acquisition timing data in parallel.

As shown in Figure 3, the time clustered cardiorespiratory encoder integrates all NMR and physiological (PD) data, (e.g. cardiac ECG and respiratory signals) that can be collected during a scan or observation period with mathematical cardiac structure/function models (M) and any data from previous time clustered cardio-respiratory encoder "scans" of the patient's heart. The result is as complete a characterization of the heart as possible, both with and without imaging. All the data is collected as quickly and as uniformly as possible, setting up an equilibrium of spin saturation and eddy currents that would make corrections for them along with magnetic field inhomogeneity easier. The integrated data is available for improving image quality or computing desired structure/ function parameters. The output of the system is either a dynamic three dimensional simulation of the heart (D) and/or maximum likelihood fitted cardiac structure-function (CF) characterization parameters ($\vec{P}$) or images (IM) improved by the reduction of flow-motion artifacts.

Referring to Figures 1 and 3, the NMR and physiological timing signals (PD) used are an NMR pulse programmer timing signal (A), the cardiac ECG R-wave (C) and analog diaphragm position respiratory signals (R). These signals are collected throughout the measurement with the microprocessor (1). These data are passed to a minicomputer (3) in which software clusters the NMR k-space profile data in the reference frame of the normalized cardiac and respiratory cycles, the C-R plane. A mathematical model (M) can be applied to the data before and during reconstruction and if desired, the model (M) can be modified with preexisting data. The data processed can be reconstructed into images (IM), which may be displayed, and/or structure/ function parameters ($\vec{P}$) can be computed. The structure/ function parameters ($\vec{P}$) can be used to drive a simulation of its beating heart. This is schematically illustrated in Figures 1 and 3.

The system is used for retrospectively clustering continuously collected NMR data with the ECG R-wave signal and digitized respiratory signal in the normalized C-R plane for the purpose of improving image quality.

The diagram shown in Fig. 4A illustrates the collection of two 64 profile images, intended to show the heart at two different phases in its cycle, with no respiratory clustering. Referring to this diagram, TCCRE clustered image A corresponding to the first 5th of the cycle, would then be formed from profiles: a2,a7,..., and e58. TCCRE clustered image B, corresponding to the fourth 5th of the cycle, would be formed from profiles a5, a9, e63, and e64. The short R2-R3 interval and long R64-R65 interval illustrate the problem with a fixed triggering delay in a variable heart cycle, profile B2 coming from the end of its R-R interval and B64 coming from the middle.

If respiratory clustering is desired, each cardiac cluster would be similarly broken down into clusters along the normalized respiratory cycle axis, say a 4 x 3 cardio-respiratory clustering, as shown in Fig. 4B.

A system to implement the method of the invention is shown in Figure 2. It includes a M68020 Single Board Computer in VME bus/power supply rack-mount chassis; a 4-8 channel ADC card (ADC); an 8 channel optically isolated digital I/O card (I/O); a digital input LATCH BOX with adjustable latching; a Digital Equipment Corp: VAX/11/750 (3) with array processor (4) based software; Philips' Flexible Reconstruction Package (FLEXIREC) for image reconstruction; and a M68020 based graphics workstation (W) with 3-D analysis and display software.

## Claims

1. A system for retrospective clustering of NMR image data of a part of a body with respect to the phases of substantially periodic cardiac and respiratory cycles comprising:

   an NMR imager (2) for collecting NMR profiles from the part of the body in a free running image data collection process, each NMR profile corresponding to a line in k-space;
   collecting means (1) for receiving cardiac R-wave time data (C) from an electrocardiogram device, respiratory diaphragm position data (R) from a respiratory position sensor, and NMR data collection timing data (A) associated with the data collection process of the NMR imager (2);
   computing means (3) suitably programmed for dividing each successive cardiac and respiratory cycle into time bins indicative of normalized phase intervals of the cardiac and respiratory cycle by using the received cardiac R-wave time data and the received diaphragm position data, and
   for sorting the collected NMR profiles into said time bins to generate a new data set of image data, comprising image data for each area of the cardio-respiratory (C-R) phase plane, the position of an NMR profile in the normalized C-R phase plane being determined by the normalized cardiac phase interval and respiratory phase interval in which it falls;

   means to fill and filter the generated data set of image data to compensate for non-uniform k-space sampling; and
   means to reconstruct a set of images (IM) from the filtered data set of image data, each image associated with one area of the normalized C-R phase plane and formed by deriving a two-dimensional or three-dimensional Fouriertransform of the image data.

2. The system of claim 1 further including:

   means to store a mathematical structure/function model (M) ;and
   means to process said data set of image data together with said mathematical model (M) while still in k-space to compute parameters (P) for driving a dynamic simulation of heart functions with said computed parameters and/or to improve the reconstructed images (IM).

3. The system of claim 2 further including means for updating the mathematical model (M) based on the reconstructed data (IM).

4. The system of claims 1,2 or 3 further including a graphic workstation (W) connected to said system, which graphic workstation (W) is suitably programmed for displaying images based on the reconstructed images (IM).

5. A method of retrospectively clustering NMR image data with respect to the phase of a substantially periodic cardiac cycle enabling in-vivo NMR imaging of a part of a body arranged in a substantially uniform, steady magnetic field, comprising the steps of :

   generating NMR profiles in the body by application of pulse sequences comprising radio frequency pulses and temporary magnetic gradient fields in a free running data collection process, each NMR profile corresponding to a line in k-space;
   collecting said NMR profiles and
   at the same time collecting digitized electrocardiogram (ECG) data of successive cardiac cycles and NMR data collection timing data;
   dividing each successive cardiac cycle into time bins indicative of normalized phase intervals of the cardiac cycle, utilizing said ECG data;
   sorting said NMR profiles into a one-dimensional array defined by the time bins of the normalized cardiac cycle, while compensating for signal to noise factors, each NMR profile in a time bin corresponding to the normalized phase interval of the cardiac cycle, during which it was collected, and selecting bins which correspond to images of moving heart tissue; the NMR profiles in a bin covering k-space incompletely and slightly differently for each bin;

filling and filtering the sorted NMR profiles in the selected bins;

reconstructing a set of images of the heart from the filtered NMR profiles, one image for each selected bin, each image formed by deriving a discrete, non-uniform Fouriertransform of the NMR profiles of the corresponding bin, to compensate for non-uniform k-space sampling.

## Patentansprüche

1. Anordnung zum retrospektiven Gruppieren von Kernspinresonanzabbildungsdaten eines Teils eines Körpers in bezug auf die Phasen im wesentlichen periodischer Herz- und Atmungszyklen mit:

einem Kernspinresonanzabbildungsgerät (2) zum Erfassen von Kernspinprofilen aus dem Teil des Körpers in einem freilaufenden Abbildungsdatenerfassungsverfahren, wobei jedes Kernspinprofil einer Zeile im k-Raum entspricht, Erfassungsmitteln (1) zum Empfangen der Herz-R-Wellenzeitdaten (C) aus einer Elektrokardiogrammanordnung, Atmungszwerchfellpositionsdaten (R) aus einem Atmungspositionssensor und Kernspindatenerfassungszeitsteuerdaten (A) in Verknüpfung mit dem Datenerfassungsverfahren des Kernspinabbildungsgeräts (2),
Berechnungsmitteln (3) geeignet programmiert zum Verteilen jedes aufeinanderfolgenden Herz- und Atmungszyklus in Zeitbins, die für normalisierte Phasenintervalle des Herz- und Ätmungszyklus durch die Verwendung der empfangenen Herz-R-Wellenzeitsteuerdaten und der empfangenen Zwerchfellpositionsdaten bezeichnend sind, und
zum Einordnen der erfaßten Kernspinprofile in die Zeitbins zum Erzeugen einer neuen Datengruppe von Abbildungsdaten mit Abbildungsdaten für jedes Gebiet der Herzzyklu/Atmungszyklusphasenebene (C-R), wobei die Position eines Kernspinprofils in der normalisierten C-R-Phasenebene von dem normalisierten Herzphasenintervall und Atmungsphasenintervall bestimmt wird, zu dem sie gehören,
Mitteln zum Ausfüllen und Filtern der erzeugten Datengruppe von Abbildungsdaten zum Ausgleichen der inhomogenen k-Raumerfassung, und
Mitteln zum Rekonstruieren einer Abbildungsgruppe (IM) aus der gefilterten Datengruppe von Abbildungsdaten, wobei jede Abbildung einem Gebiet der normalisierten C-R-Phasenebene zugeordnet wird und durch Ableiten einer zweidimensionalen oder dreidimensionalen Fourier-Transformation der Abbildungsdaten gebildet wird.

2. System nach Anspruch 1 außerdem mit:

Mitteln zum Speichern eines mathematischen Struktur/Aufgabenmodells (M) und
Mitteln zum Bearbeiten der Datengruppe der Abbildungsdaten zusammen mit dem mathematischen noch im k-Raum befindlichen Modell (M) zum Berechnen der Parameter (P) zum Ansteuern einer dynamischen Simulierung der Herzaufgaben mit den berechneten Parametern und/oder zur Verbesserung der rekonsturierten Bilder (IM).

3. System nach Anspruch 2 außerdem mit Mitteln zum Aktualisieren des mathematischen Modells (M), das auf den rekonstruierten Daten (IM) basiert.

4. System nach Anspruch 1, 2 oder 3 außerdem mit einer graphischen Arbeitsstation (W), die mit dem System verbunden ist, wobei die graphische Arbeitsstation (W) auf geeignete Weise zum Wiedergeben von Abbildungen programmiert ist, die auf den rekonstruierten Abbildungen (IM) basieren.

5. Verfahren zum retrospektiven Gruppieren von Kernspinabbildungsdaten in bezug auf die Phase eines im wesentlichen periodischen Herzzyklus zur Ermöglichung der In-Vivo-Kernspinabbildung eines Teiles eines in einem im wesentlichen homogenen statischen Magnetfeld angeordneten Körpers mit folgenden Schritten:

das Erzeugen von Kernspinprofilen im Körper durch Zufuhr von Impulssequenzen mit Hr-Impulsen und zeitlichen Magnetgradientenfeldern in einem freilaufenden Datenerfassungsverfahren, wobei jedes Kernspinprofil einer Zeile im k-Raum entspricht,
das Erfassen der Kernspinprofile und
das gleichzeitige Erfassen digitalisierter Elektrokardiogrammdaten (EKG) aufeinanderfolgender Herzzyklen und Kernspindatenerfassungszeitsteuerdaten,
das Aufteilen jedes aufeinanderfolgenden Herzzyklus in Zeitbins, die für normalisierte Phasenintervalle des Herzzyklus unter Verwendung der EKG-Daten bezeichnend sind,
das Einordnen der Kernspinprofile in ein eindimensionales Feld, das von den Zeitbins des normalisierten Herzzyklus definiert ist, während Signal/Rauschfaktoren ausgeglichen werden, und jedes Kernspinprofil in einem Zeitbin dem normalisierten Phasenintervall des Herzzyklus entspricht, in dem es erfaßt wurde, und das Auswählen von Bins, die Abbildungen des beweglichen Herzgewebes entsprechen, wobei die Kernspinprofile in einem Bin den k-Raum unvollständig und etwas abweichend für jedes Bin überdecken,

das Ausfüllen und Filtern der eingeordneten Kernspinprofile in den ausgewählten Bins,

das Rekonstruieren einer Gruppe von Abbildungen des Herzens aus den gefilterten Kernspinprofilen, wobei es eine Abbildung für jedes gewählte Bin gibt, und jede Abbildung durch Ableiten einer diskreten inhomogenen Fourier-Transformation der Kernspinprofile des entsprechenden Bins gebildet wird, um inhomogene k-Raumabtastung auszugleichen.

## Revendications

1. Système pour le groupement rétrospectif de données d'image RMN d'une partie d'un corps avec référence aux phases de cycles cardiaques et respiratoires sensiblement périodiques, comprenant :

un appareil d'imagerie RMN (2) pour collecter les profils de RMN de la partie du corps dans un processus de collecte de données d'image en mode relaxé, chaque profil de RMN correspondant à une ligne dans l'espace k;

des moyens de collecte (1) pour recevoir des données de temps d'onde R cardiaque (C) d'un dispositif à électrocardiogramme, des données de position du diaphragme respiratoire (R) d'un capteur de position respiratoire et des données de synchronisation de collecte de données RMN (A) associés au processus de collecte de données de l'appareil d'imagerie RMN (2);

des moyens de calcul (3) convenablement programmés pour diviser chaque cycle cardiaque et respiratoire successifs en compartiments de temps représentatifs d'intervalles de phase normalisés du cycle cardiaque et respiratoire en utilisant les données de temps d'onde R cardiaque reçues et les données de position de diaphragme reçues;

pour répartir par triage les profils de RMN collectés dans les compartiments de temps pour engendrer un nouveau jeu de données d'image comprenant des données d'image pour chaque aire du plan de phase cardio-respiratoire (CR), la position d'un profil de RMN dans le plan de phase CR normalisé étant déterminée par l'intervalle de phase cardiaque normalisé et l'intervalle de phase respiratoire normalisé auquel il appartient;

des moyens pour compléter et filtrer le jeu de données d'image obtenu afin de compenser l'échantillonnage non uniforme de l'espace k, et

des moyens pour reconstruire un jeu d'image (IM) à partir du jeu de données d'image filtré, chaque image étant associée à une aire du plan de phase (CR) normalisé et étant formée par dérivation d'une transformée de Fourier bidimensionnelle ou tridimensionnelle des données d'image.

2. Système suivant la revendication 1, comprenant, de plus :

des moyens pour mémoriser un modèle structure/fonction mathématique (M); et

des moyens pour traiter ledit jeu de données d'image conjointement avec le modèle mathématique (M), encore dans l'espace k, pour calculer les paramètres (P) pour créer une simulation dynamique des fonctions cardiaques à l'aide de ces paramètres calculés et/ou pour améliorer les images reconstruites (IM).

3. Système suivant la revendication 2, comprenant, de plus, des moyens pour mettre à jour le modèle mathématique (M) basé sur les données reconstruites (IM).

4. Système suivant la revendication 1, 2 ou 3, comprenant, en outre, un poste de travail graphique (W) connecté à ce système, lequel poste de travail graphique (W) est convenablement programmé pour afficher des images basées sur les images reconstruites (IM).

5. Procédé pour grouper rétrospectivement des données d'image RMN en fonction de la phase d'un cycle cardiaque sensiblement périodique permettant l'imagerie RMN in vivo d'une partie d'un corps disposée dans un champ magnétique statique sensiblement uniforme, comprenant les étapes consistant à:

produire des profils RMN dans le corps par l'application de séquences d'impulsions comprenant des impulsions de radiofréquence et des champs de gradient magnétiques temporaires au cours d'un processus de collecte de données en mode relaxé, chaque profil RMN correspondant à une ligne dans l'espace k;

collecter les profils de RMN et simultanément collecter les données d'électrocardiogramme (ECG) numérisées de cycles cardiaques successifs et les données de synchronisation de collecte de données de RMN;

diviser chaque cycle cardiaque successif en compartiments de temps représentatifs des intervalles de phase normalisés du cycle cardiaque au moyen de ces données d'ECG;

répartir par triage ces profils de RMN dans un agencement unidimensionnel défini par les compartiments de temps du cycle cardiaque normalisé tout en compensant les facteurs signal:bruit, chaque profil de RMN dans un compartiment de temps correspondant à l'intervalle de phase normalisé du cycle cardiaque, pendant lequel il a été collecté et sélectionner les compartiments qui correspondent à des images du tissu cardiaque en mouvement, les profils de RMN dans un compartiment couvrant l'espace k incomplètement et un peu différemment pour chaque compartiment;

compléter et filtrer les profils de RMN triés dans les compartiments sélectionnés;

reconstruire un jeu d'images du coeur à partir des profils RMN filtrés, avec une image pour chaque compartiment sélectionné, chaque image étant formée par dérivation d'une transformée de Fourier non uniforme discrète des profils de RMN du compartiment correspondant afin de compenser l'échantillonnage non uniforme de l'espace k.

FIG.1

FIG.2

## FIG.3

## FIG.4A

## FIG.4B